(19) Europäisches Patentamt / European Patent Office / Office européen des brevets

(11) **EP 3 055 800 B1**

(12) **EUROPEAN PATENT SPECIFICATION**

(45) Date of publication and mention
of the grant of the patent:
**30.10.2019  Bulletin 2019/44**

(51) Int Cl.:
**G16H 20/10** *(2018.01)*

(21) Application number: **14786401.1**

(22) Date of filing: **30.09.2014**

(86) International application number:
**PCT/US2014/058186**

(87) International publication number:
**WO 2015/053979 (16.04.2015 Gazette 2015/15)**

(54) **SURGICAL AND POST-SURGICAL FLUID MANAGEMENT TO PREVENT LOW HEMATOCRIT**

CHIRURGISCHES UND POSTCHIRURGISCHES FLÜSSIGKEITSMANAGEMENT ZUR
VERHINDERUNG NIEDRIGER HÄMATOKRITWERTE

GESTION DE FLUIDE CHIRURGICAL ET POST-CHIRURGICAL POUR PRÉVENIR UNE FAIBLE
HÉMATOCRITE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.10.2013   US 201314049453**

(43) Date of publication of application:
**17.08.2016   Bulletin 2016/33**

(73) Proprietor: **Medtronic Inc.
Minneapolis, MN 55432 (US)**

(72) Inventor: **BEARSS, Mark
Santa Rosa, California 95403 (US)**

(74) Representative: **Zimmermann & Partner
Patentanwälte mbB
Josephspitalstr. 15
80331 München (DE)**

(56) References cited:
**EP-A1- 2 581 846       US-A- 5 984 893
US-A1- 2010 152 544**

• **CORMACK J E ET AL: "Hematocrit prediction and
preservation for cardiopulmonary bypass",
INTERNET CITATION, 16 June 2003 (2003-06-16),
XP009102385, Retrieved from the Internet:
URL:http://perfline.com/ejournal/2002/jec0
102.html [retrieved on 2008-06-27]**
• **CH AUER ET AL: "Blut und Blutkomponenten als
Primevolumen der extrakorporalen Zirkulation
bei Säuglingen Grundlagen zur Linzer
Perfusionsführung", KARDIOTECHNIK, vol. 13,
no. 4, 1 December 2004 (2004-12-01), pages
102-108, XP055167738, ISSN: 0941-2670**

**Description**

**FIELD**

This disclosure generally relates to, among other things, devices, systems and methods for managing patient blood fluid volume and hematocrit before, during or after surgery, such as cardiopulmonary bypass.

**BACKGROUND**

Hemodilution can lead to a number of perioperative and postoperative morbidities and mortality. For example, hemodilution can result in inadequate oxygen delivery and ischemic organ injury, renal failure, heart failure, etc. During procedures such as cardiopulmonary bypass (CPB), a patient is connected to equipment having fluid circuitry that is typically primed with a red blood cell (RBC)-free crystalloid solution. The volume of the circuitry primed with RBC-free solution serves to dilute the hematocrit of the patient's blood.

Accordingly, a patient's blood fluid volume and hematocrit levels are typically determined prior to undergoing CPB. In addition to the patient's blood fluid volume, the volume of the CPB circuitry and the volume of anesthesia or other IV fluids expected to be delivered is determined. A calculation may then be made to determine the predicted on-pump hematocrit, accounting for dilution by the CPB circuitry and IV volume.

If the predicted on-pump hematocrit is below a prescribed level, a plan may be implemented to reduce hemodilution before beginning CPB. Measures towards reducing hemodiluition include (1) minimizing volume added from IV; (2) shortening lines to reduce prime volume; (3) adding blood products to the CPB circuit; (4) incorporating a technique that displaces the crystalloid prime using the patient's blood before initiating CPB (e.g., "autologous priming"); and (5) adding hematoconcentrator to the circuit to extract water from the circulating blood.

During CPB, the hydration status of the patient and circulating blood volume in the CPB circuit and the patient are in a state of flux. For example, intravascular water may migrate to extravasculature or "third-space" compartments; additional crystalloid solution may be added by a perfusionist to the CPB circuit to maintain a minimum venous reservoir volume; cardioplegia solution and medications may be added to the CPB circuit; additional fluids and medications may be administered via IV lines; intravascular volume loss may occur via the kidneys and urinary output; patient blood volume and red cell volume loss may occur due to vascular bleeding; insensible water loss can occur; patient metabolism may reduce the hydration status; and the like.

EP 2 581 846 A1 relates to a cardiopulmonary bypass or CPB monitoring tool. US 2010/152544 A1 relates to a cardiac monitoring system and method. US 5,984,893 A relates to a blood infusion control system.

During CPB, hematocrit levels are not typically recalculated to account for one or more of the changes that may occur to fluid and red blood cell levels, but real-time changes in hematocrit may be monitored via an in-line monitor. While useful, real-time monitoring of hematocrit levels tends to result in a reactionary plan (hematocrit levels determined after a change has occurred) rather than a predictive plan (understanding how a change may affect hematocrit) to prevent excessive hemodilution during CPB. Further, in-line hematocrit monitoring is typically only performed during the CPB procedure, not during post-operative recovery where blood draws and lab tests are typically performed to monitor hematocrit.

**SUMMARY**

In embodiments, this disclosure describes, among other things, devices, systems and methods for patient fluid management that are more predictive than reactionary. In embodiments, the fluid capacity of a patient; *i.e.,* the amount of hematocrit free fluid that can be added to the system (patient, CPB circuitry, etc.) to avoid reaching, or to just reach but not fall below, a predetermined low hematocrit limit, is determined. The fluid capacity or amount of fluid to avoid reaching, or to just reach but not fall below, a predetermined low hematocrit threshold may be displayed, so that a member of the surgical team, such as a surgeon, perfusionist, or anesthesiologist, can better understand the fluid capacity of the patient and better understand the effect that adding fluid will have on the patient's fluid capacity. That is, members of the surgical team will better understand whether adding fluid to the system will result in undesired or excessive hemodilution before the fluid is added.

The devices, systems and methods described herein, in various embodiments, take into account whether fluid added or lost from a patient is RBC-free or contains RBCs to adjust for overall hematocrit levels within the patient system. The devices, systems and methods described herein may be used during surgery or post-surgical recovery, may be used as a teaching tool, or the like. In embodiments, the devices, systems and methods described herein may be used to determine what effects fluid introduction or loss may have on hematocrit or fluid capacity before the fluid is introduced to, or withdrawn from, the patient.

A computer-implemented method according to claim 1 is claimed herein. An exemplary method includes receiving input regarding the weight of a patient; receiving input regarding hematocrit of blood of the patient; setting a lower hematocrit level for the patient; and calculating a volume of hematocrit-free fluid that the patient can receive into a blood volume compartment of the patient until the lower hematocrit level is reached.

Non-transitory computer-readable media that, when implemented, cause a device to carry out one or more methods described herein are also described. Devices employing such non-transitory computer-readable media or otherwise configured to carry out the method are also described.

One or more embodiments of the devices, systems and methods described herein have one or more advantages relative to prior devices, systems and methods for fluid management associated with surgery. Those of skill in the art, upon reading the present disclosure and accompanying drawings, will readily appreciate these advantages.

## BRIEF DESCRIPTION OF THE DRAWINGS

**FIG. 1** is a schematic diagram of a patient during cardiopulmonary bypass (CPB) illustrating some interactions that affect patient blood compartment volume.

**FIGS. 2-5** are flow diagrams illustrating embodiments of methods, or portions thereof, as described herein.

**FIG. 6** is a schematic block diagram of a system for carrying out the methods described herein.

**FIGS. 7A-D** are schematic drawings of prophetic screen shots of a device configured to carry out an embodiment of a method described herein.

**FIGS. 8A-C** are schematic drawings of prophetic screen shots of a device configured to carry out an embodiment of a method described herein.

**FIG. 9** is a schematic drawing of a prophetic screen shot of a device configured to carry out an embodiment of a method described herein.

**FIGS. 10A-E** are schematic drawings of prophetic screen shots of a device configured to carry out an embodiment of a method described herein.

The schematic drawings are not necessarily to scale. Like numbers used in the figures refer to like components, steps and the like. However, it will be understood that the use of a number to refer to a component in a given figure is not intended to limit the component in another figure labeled with the same number. In addition, the use of different numbers to refer to components is not intended to indicate that the different numbered components cannot be the same or similar.

## DETAILED DESCRIPTION

In the following detailed description several specific embodiments of compounds, compositions, apparatuses, systems and methods are disclosed. It is to be understood that other embodiments are contemplated and may be made without departing from the scope of the present disclosure. The following detailed description, therefore, is not to be taken in a limiting sense.

This disclosure generally relates to, *inter alia,* patient fluid management before, during or after surgeries in which hemodilution may be a concern, such as CPB. Rather than providing only information regarding hematocrit levels of blood of a patient, the present disclosure describes methods, devices and systems that provide information regarding the fluid capacity of a patient. As used herein, "fluid capacity of a patient" is the amount of hematocrit-free fluid that can be added to a blood compartment of a patient to avoid reaching, or to just reach but not fall below, a user defined low hematocrit limit. The fluid capacity of the patient may be displayed to allow a health care provider to make decisions regarding effects of addition of fluid to the blood compartment of the patient. In embodiments, the methods, devices and systems described herein inform a health care provider, such as a member of a surgical team, of the maximum amount of a given fluid, whether containing red blood cells or hematocrit-free, that can be added to the blood compartment to avoid reaching, or to just reach but not fall below, the user-defined low hematocrit limit. In embodiments, the methods, systems and devices described herein can track the fluid status of a blood compartment of the patient over time and can provide updates regarding the fluid capacity of the patient in light of the changing fluid status.

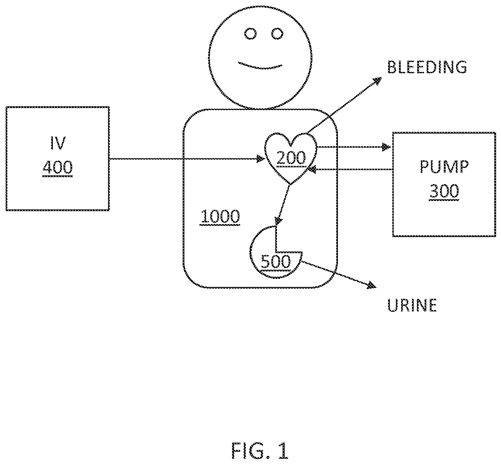

Referring now to **FIG. 1**, a schematic drawing of a patient **1000** during a CPB surgery is shown. The patient **1000** has a blood fluid compartment **200** through which blood circulates. The blood fluid compartment **200** of the patient is operably coupled to an external pump **300** through, for example, tubular conduits so that the patient's blood may be circulated by pump **300** during CPB. One or more IV apparatuses **400** are also operably coupled with the blood compartment **200** for intravenous (IV) delivery of one or more therapeutic agents, such as anesthetics, cardioplegia solutions or medications, etc., to the patient **1000**. In the depicted embodiment, the surgical blood compartment of the patient can be considered to be the native blood compartment of the patient **200** and those portions of the pump **300**, IV apparatus **400** and associated lines that are in communication with the native blood compartment of the patient **200**. Accordingly, the volume of the blood compartment of the patient during surgery is the volume of the native blood compartment of the patient **200** and the fluid volume contained by the pump **300**, IV apparatus **400** and associated lines.

Also depicted in **FIG. 1** are some of the ways in which the volume of fluid in a patient's blood fluid compartment may change. For example, the patient may lose hematocrit-containing fluid through bleeding or may lose relatively hematocrit-free fluid through urinary excretion. Of course, these are just some examples of how fluid status of the patient may change over time. Other examples include intravascular water migrating to extravasculature or "third-space" compartments; insensible water loss; patient metabolism reducing the hydration status; and the like.

Monitoring and accounting for such fluid status changes, particularly with regard to fluid capacity of the patient, can be challenging, and is not currently being done. The methods, devices and systems described herein, in various embodiments, account for such changes and provide predictive information that may be helpful to members of a surgical team or post-surgical health care provider.

In many aspects, the methods described herein include determining the patient blood volume (PVB), which is the volume of the blood compartment. The volume of the native blood compartment of the patient **200** can be roughly determined by multiplying the patient's weight by a conversion factor. The conversion factor represents a volume of blood per unit of mass of the body weight of the patient. For example, a conversion factor of 65 to 70 mL of blood per kilogram of body weight may be used. By way of example, if a patient weighs 75 kilograms and a conversion factor of 70 mL/kg is used, the PVB is 5,250 mL (75 kg x 70 mL/kg = 5,250 mL).

If the hematocrit of the patient's blood is known, the red blood cell volume (RCV) can be determined by multiplying the PVB by the hematocrit. By way of example, if the patient's PVB is 5,250 mL and the hematocrit is 38%, the RCV is 1,995 mL (5,250 mL x 0.38 = 1,995 mL). Typically, the patient's hematocrit is determined prior to CPB and can be used for baseline calculations of RCV.

Once RCV is known, the hematocrit level of the blood once the patient is hooked up to the pump **300** and IV apparatus **400** is determined by dividing the RCV by the sum of the PBV, the prime volume of the pump **300** and the IV volume. By way of example, the surgical or "on-pump" hematocrit of a patient having a RCV of 1,995 mL and a PVB of 5,250 mL, a pump prime volume of 1200 mL, and an IV volume of 800 mL is 0.275 or 27.5 % [1,995 mL/(5,250 mL + 1200 mL + 800 mL) = .275]. The prime volume of the pump may be determined empirically by determining the total volume of crystalloid solution needed to prime the pump and lines, by using manufacturer specifications regarding pump and line prime volumes, or the like. The IV volume may be determined by the volume of IV fluids that an anesthesiologist or other healthcare provider operably couples to the patient's blood compartment.

The on-pump hematocrit may be used to aid in blood fluid management decisions prior to surgery, such as minimizing IV volumes, shortening pump lines to reduce prime volume, add blood products to the circuit, employ autologous priming, add hemoconcentrator to the circuit, and the like. However, these decisions are only helpful at a point in time prior to beginning of CPB and may not be as valuable as the fluid status of the patient changes during surgery; e.g., additional crystalloid solution being added by a perfusionist to the CPB circuit to maintain a minimum venous reservoir volume; cardioplegia solutions or medications or other medications being introduced via IV lines; water loss, bleeding, and the like.

Managing a patient's hemodilution status is generally considered to be important during cardiac surgery, particularly when a patient is on CPB. One strategy that is commonly practiced involves maintaining the hematocrit above a user-defined low-limit hematocrit level when a patient is on cardiopulmonary bypass. Another protocol limits giving a patient allogeneic blood unless the hematocrit reaches a user-defined "transfusion trigger". This is a practice that would be adhered to during the patient's entire continuum of care, with an overarching goal to avoid allogeneic transfusions.

While inline hematocrit monitoring devices do offer utility for tracking and trending changes in hemodilution, they are only used during CPB and they only display a change in hematocrit after crystalloid solution or blood products are administered to the patient. Periodic blood-lab analysis of hematocrit also represents results after events occur that impacts a patient's fluid balance. There is currently no tool or device that displays pre-emptive information relative to fluid management protocols.

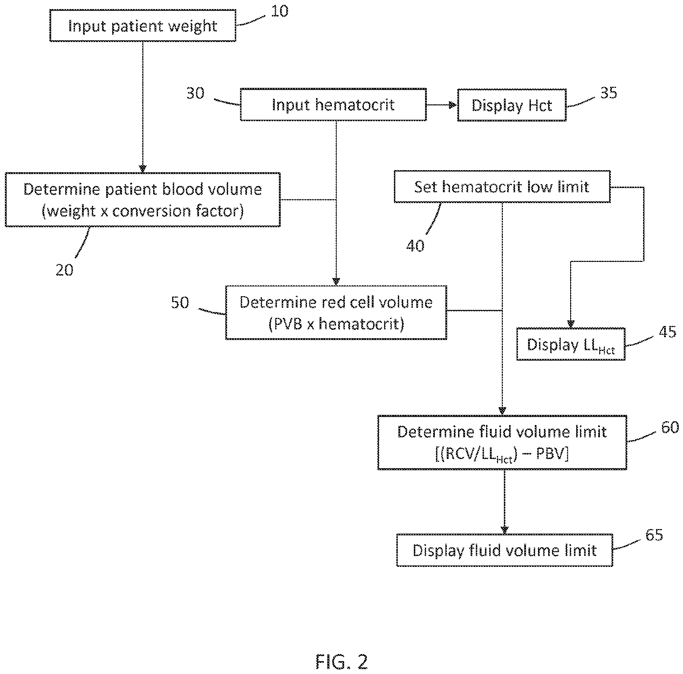

Referring now to **FIG. 2,** a method that may be carried out by a device or system to provide more pre-emptive information is shown. The method includes receiving input regarding the weight of a patient (**10**) and determining the patient's blood volume (**20**); e.g., as described above (PVB = weight x conversion factor). The method also includes receiving input regarding hematocrit levels of the patient's blood (**30**) and determining the red blood cell volume (**50**); e.g., as described above (RCV = PVB x hematocrit). The method further includes setting a low hematocrit limit (**40**) and determining a fluid volume limit (**60**). The fluid volume limit (FVL) may be determined by subtracting the PVB from the RCV divided by the set hematocrit low limit ($LL_{Hct}$). That is, FVL = (RCV/$LL_{Hct}$) - PBV. The FVL is the volume of hematocrit-free fluid that may be added to the CPB circuit, which includes the PVB, the pump prime volume, and the IV volume, to avoid reaching, or to just reach but not fall below, the user-defined hematocrit low limit.

The hematocrit low limit may be set at any suitable low limit. In many cases, a healthcare provider, such as a surgeon, inputs a low hematocrit limit to set the limit. In many instances, the hematocrit low limit may be set at 0.24 or 24%. Of course, other low limits may be set as deemed appropriate by an attending health care provider. In embodiments, a device carrying out the method depicted in **FIG. 2** may set the hematocrit low limit as a default limit if a healthcare provider does not enter a hematocrit low limit.

Hematocrit levels may be input at step **30** by a healthcare provider after reviewing results or lab tests, viewing an in-line hematocrit sensor, or the like. Alternatively, a system or device carrying out the method depicted in **FIG. 2** may include or be in communication with a hematocrit sensor to directly receive hematocrit readings in real-time or near real-time.

The method depicted in **FIG. 2** further includes displaying the fluid volume limit (**65**). By displaying the FVL, a healthcare provider such as a member of the surgical or post-surgical team will know how much RBC-free fluid can be introduced into the patient's blood compartment to avoid reaching, or to just reach but not fall below, the user defined low hematocrit limit.

The low hematocrit limit may also be displayed (**45**). The hematocrit level within the blood compartment may also be displayed (**35**). As discussed above, actual hematocrit may be obtained through an in-line hematocrit sensor, results of lab testing, or the like.

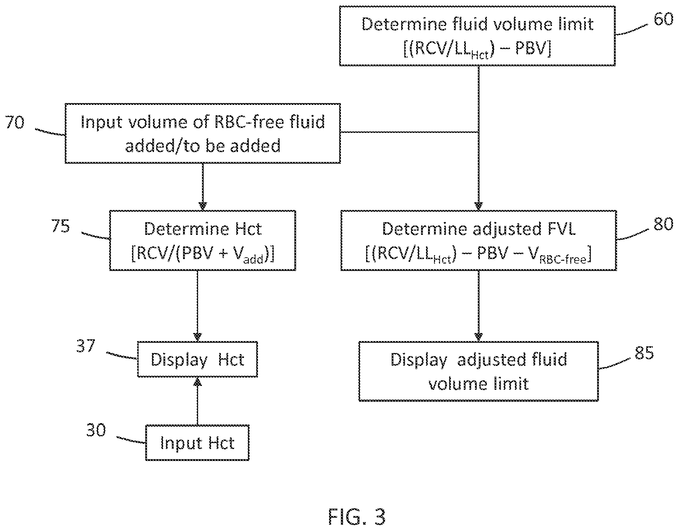

Referring now to **FIG. 3,** a method for adjusting the FVL and hematocrit (Hct) when red blood cell (RBC)-free solution is added to, or removed or lost from, the patient's blood compartment is shown. The method in **FIG. 3** may be useful to determine the FVL and Hct when accounting for pump prime volume and IV volume prior to or at the beginning of surgery. Of course, the method may also be useful during or after surgery when RBC-free solution is added or lost. Times when RBC-free fluid may be added or lost during or after surgery include when a perfusionist adds additional crystalloid solution to the CPB circuit, when additional medications or fluids are added via IV lines, when the patient loses fluid due to urinary output, and the like.

The method depicted in **FIG. 3** may be carried out on its own or may be a continuation of the method in **FIG. 2**. As shown in **FIG. 3**, a starting point for fluid volume limit (FVL) is known, which may be obtained from the method depicted in **FIG. 2**. In any case, the FVL may be determined from red blood cell volume (RCV), hematocrit low limit ($LL_{Hct}$) and patient blood volume (PBV), which may include, among other things, the pump prime volume and the IV volume. The calculations for determining FVL may be carried out generally as described above with regard to **FIG. 2.** In addition to determining (e.g., calculating or inputting) the initial FVL (**60**), the method of **FIG. 3** includes inputting the volume of RBC-free fluid added or to be added to the patient blood compartment (**70**) and determining an adjusted FVL (**80**) based on the volume of RBC-free fluid added or to be added. In will be understood that the volume of RBC-free fluid added may be a loss of RBC-fluid (i.e., the volume of fluid added will be negative).

The adjusted FVL may be determined by subtracting the volume of RBC-free fluid added or to be added and the PBV from the RCV divided by the $LL_{Hct}$ (i.e., FVL = RCV/$LL_{Hct}$ - PBV - $V_{RBC-free}$). The adjusted FVL may then be displayed (**85**) to allow a healthcare provider to know how much additional RBC-free fluid can be introduced into the patient's blood compartment to avoid reaching, or to just reach but not fall below, the user defined low hematocrit limit, following the addition of the RBC-free fluid at step **70**.

As shown in **FIG. 3,** the effect of the addition of the RBC-free fluid on Hct may also be determined (**75**). For example, Hct that will result after addition of the RBC-free fluid may be calculated by dividing the RCV by the sum of the PBV and the volume of RBC-free fluid added [i.e., Hct = RCV/(PBV + $V_{add}$)], where the PBV is the PBV prior to the addition of the RBC-free fluid. The calculated Hct may be displayed (**37**). Alternatively, or in addition, input Hct (**30**) may be displayed (**37**). Input Hct may be Hct obtained from an in-line sensor, lab tests, or the like, as discussed above with regard to **FIG. 2.**

A healthcare provider may choose to employ a device or system to carry out the method depicted in **FIG. 3** to determine what will happen to FVL and Hct prior to adding the RCB-free fluid to determine whether parameters regarding the fluid addition should be modified before adding the fluid. In this manner, the healthcare provider can make a better informed decision as to how to proceed relative to prior more reactionary procedures (add the fluid and react to what happens with hematocrit).

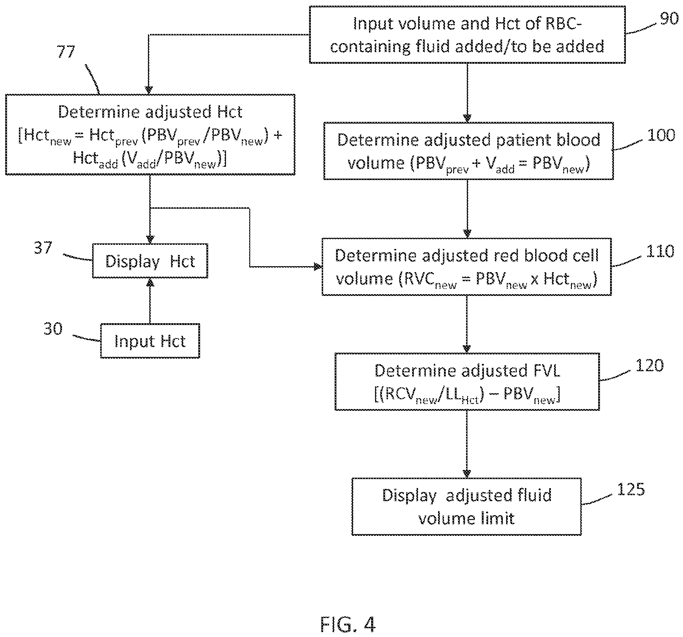

Referring now to **FIG. 4**, a method for adjusting the FVL and Hct when RBC-containing fluid is added to, or removed or lost from, the patient's blood compartment is shown. The method depicted in **FIG. 4** assumes that the PVB and Hct are known prior to adding the RBC-containing fluid. The initial PVB and Hct may be obtained or determined as described above with regard to **FIG. 2** or **FIG. 3**. The method depicted in **FIG. 4** may be carried out on its own or may be a continuation or part of the method depicted in, and discussed with regard to, **FIG. 2** or the method depicted in, and discussed with regard to, **FIG. 3**.

As with the method depicted in **FIG. 3**, the method depicted in **FIG. 4** assumes a starting point for patient blood volume (PVB), which may include pump prime volume, IV volume, etc., and hematocrit (Hct) is known. The method depicted in **FIG. 4** includes inputting a volume and Hct of RBC-containing fluid added or to be added (**90**). It will be understood that the fluid added or to be added may be fluid lost, such as through post-operative bleeding, in which case the volume to be added will be a negative volume. If the fluid loss is due to bleeding, the hematocrit of the fluid loss should equal the hematocrit of the blood within the PVB. Of course, the Hct of the blood lost may be tested to verify the Hct.

Based on the previously known PVB and Hct, an adjusted PVB may be determined (**100**) and an adjusted Hct may be determined (**77**), taking into account the volume and Hct of the fluid added or to be added. Adjusted PVB can be calculated by adding the volume of the fluid added to the previous PVB ($PVB_{new} = PVB_{prev} + V_{add}$). Adjusted Hct can be determined by adding (i) the previous Hct in the patient's blood compartment times $PVB_{prev}$ dividided by $PVB_{new}$; and (ii) the Hct of the fluid to be added times the volume of the fluid to be added divided by $PVB_{new}$ [$Hct_{new} = Hct_{prev}$ ($PVB_{prev}/PVB_{new}$) + $Hct_{add}$ ($V_{add}/PVB_{new}$)].

Based on the adjusted Hct and the adjusted PVB, the adjusted red blood cell volume (RCV) may be determined (**110**) by multiplying the adjusted PVB by the adjusted Hct ($RCV_{new} = PVB_{new} \times Hct_{new}$). The adjusted fluid volume limit (FVL) can then be determined (**120**) by subtracting the adjusted PVB from the adjusted RCV divided by the hematocrit low limit [$FVL_{new} = (RCV_{new}/LL_{Hct}) - PVB_{new}$]. The adjusted FVL may then be displayed (**125**) to allow a healthcare provider to know how much additional RBC-free fluid can be introduced into the patient's blood compartment to avoid reaching, or to just reach but not fall below, the used-defined low hematocrit limit, following the addition of the RBC-free fluid at step **70.**

As shown in **FIG. 4**, the calculated Hct may be displayed (**37**). Alternatively, or in addition, input Hct (**30**) may be displayed (**37**). Input Hct may be Hct obtained from an in-line sensor, lab tests, or the like, as discussed above with regard to **FIGS. 2-3.**

A healthcare provider may choose to employ a device or system to carry out the method depicted in **FIG. 4** to determine what will happen to FVL and Hct prior to adding the RCB-containing fluid to determine whether parameters regarding the fluid addition should be modified before adding the fluid. In this manner, the healthcare provider can make a better informed decision as to how to proceed relative to prior more reactionary procedures (add the fluid and react to what happens with hematocrit).

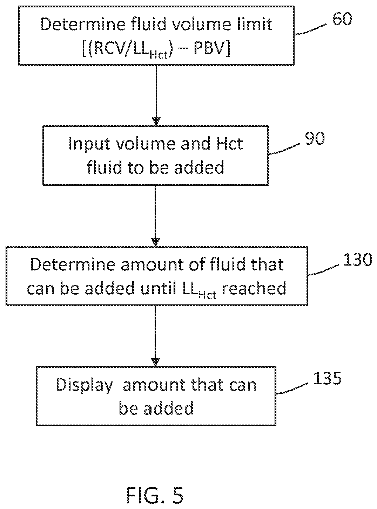

By way of example and with reference to **FIG. 5,** a method is shown that includes determining the FVL (**60**), inputting volume and Hct of fluid to be added (**90**), determining the amount of fluid that can be added until the hematocrit low limit ($LL_{Hct}$) is reached (**130**), and displaying the amount of the fluid that can be added to avoid reaching, or to just reach but not fall below, the limit (**135**). A healthcare provider can use the information regarding the volume to avoid reaching the $LL_{Hct}$ to better understand the affect that adding the fluid will have and whether adjustments to the fluid parameters should be made prior to adding the fluid. For example, the healthcare provider may decide that additional RBCs should be added to the fluid prior to introduction to the patient's blood compartment. The healthcare provider may run a similar analysis on other volumes and hematocrit levels to determine which combination of volume and hematocrit level will best serve the patient's needs prior to introducing the fluid to the blood compartment.

The FVL may be determined or input as discussed above with regard to **FIGS. 2-4**; e.g., $FVL = (RCV/LL_{Hct}) - PVB$. The amount of fluid that can be introduced to avoid reaching, or to just reach but not fall below, the $LL_{Hct}$ may be calculated in any suitable manner. For example, calculations similar to those discussed above with regard to **FIG. 4** may be used. For example, the volume of fluid that can be added ($V_{add}$) to just reach but not fall below the hematocrit low limit can be calculated by subtracting the product of the hematocrit low limit ($LL_{HCT}$) and the previous patient blood volume ($PVB_{prev}$) from the product of the previous hematocrit ($Hct_{prev}$) and the $PVB_{prev}$ and dividing the resulting value by $LL_{HCT}$ minus the hematocrit of the fluid to be added ($Hct_{add}$). That is, $V_{add} = [(Hct_{prev} - LL_{HCT})(PVB_{prev})]/(LL_{HCT} - HCT_{add})$.

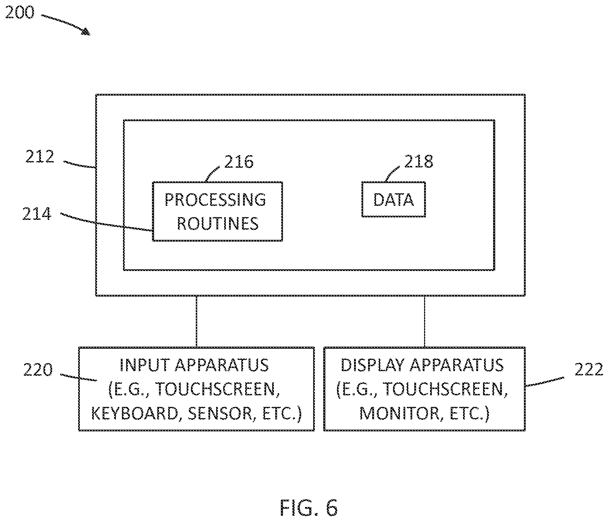

A schematic drawing of an embodiment of a system **200** depicted in **FIG. 6** may be used to execute embodiments of the methods or processes described herein (e.g., the methods depicted in, and discussed with regard to, **FIGS. 2-5** or portions or combinations thereof). In embodiments, the system **200** is a CPB pump apparatus. The system **200** could, for example, alternatively include a desktop computer, a laptop computer, a tablet device, or the like.

In the embodiment depicted in **FIG. 6**, the system **200** includes computing apparatus **212**. The computing apparatus **212** may be configured to receive input from input apparatus **220** and transmit output to display apparatus **222**. Further, the computing apparatus **212** may include data storage **214**. Data storage **214** may allow for access to processing programs or routines **216** and one or more other types of data **218** that may be employed to carry out the methods or processes described herein. For example, the computing apparatus **212** may be configured to calculate PVB, RCV, Hct, FVL, or the like (e.g., as described above with regard to **FIGS. 2-5**) and display status information on the regarding the PVB, RCV, Hct, FVL, or the like on display apparatus **222.**

The computing apparatus **212** may be operatively coupled to the input apparatus **220** and the display apparatus **222** to, e.g., transmit data to and from each of the input apparatus **220** and the display apparatus **222.** For example, the computing apparatus **212** may be electrically coupled to each of the input apparatus **220** and the display apparatus **222** using, e.g., analog electrical connections, digital electrical connections, wireless connections, bus-based connections, etc. As described further herein, a user may provide input to the input apparatus **220** to manipulate, or modify, one or more graphical depictions displayed on the display apparatus **222**.

Further, various devices and apparatuses may be operatively coupled to the computing apparatus **212** to be used with the computing apparatus **212** to perform one or more of the methods, processes or logic described herein. As shown, the system **200** may include input apparatus **220,** display apparatus **222**. If the system **200** includes a CPB pump apparatus (not shown in **FIG. 6**), the CPB pump apparatus may be operably coupled to the computing apparatus **212.**

The input apparatus **220** may include any apparatus capable of providing input to the computing apparatus **212** to perform the functionality, methods, processes or logic described herein. For example, the input apparatus **220** may include a touchscreen (e.g., capacitive touchscreen, a resistive touchscreen, a multi-touch touchscreen, etc.), a mouse, a keyboard, a trackball, hematocrit sensor, etc. The input apparatus **220** may allow a user to select and view various status information corresponding to the fluid status of a patient when used in conjunction with the display apparatus **222** (e.g., displaying a graphical user interface).

Likewise, the display apparatus **222** may include any apparatus capable of displaying information to a user, such as a graphical user interface, etc., to perform the functionality, methods, processes or logic described herein. For example, the display apparatus **222** may include a liquid crystal display, an organic light-emitting diode screen, a touchscreen, a cathode ray tube display, etc. As described further herein, the display apparatus **222** may be configured to display a graphical user interface that includes one or more regions such as a patient fluid status region including status information corresponding to one or more parameters of patient fluid management (e.g., PVB, RVC, Hct, FVL, etc.). As used herein, a "region" of a graphical user interface may be defined as a portion of the graphical user interface within which information may be displayed or functionality may be performed. Regions may exist within other regions, which may be displayed separately or simultaneously. For example, smaller regions may be located within larger regions, regions may be located side-by-side, etc. Additionally, as used herein, an "area" of a graphical user interface may be defined as a portion of the graphical user interface located with a region that is smaller than the region it is located within.

The processing programs or routines **216** may include programs or routines for performing computational mathematics, matrix mathematics, standardization algorithms, comparison algorithms, or any other processing required to implement one or more methods or processes, or portions or combinations thereof, described herein. Data **218** may include, for example, patient weight data, hematocrit data, hematocrit conversion factors, patient fluid volume, red blood cell volume, hematocrit low limit, notifications, graphics (e.g., graphical elements, icons, buttons, windows, dialogs, pull-down menus, graphic areas, graphic regions, 3D graphics, etc.), graphical user interfaces, results from one or more processing programs or routines employed according to the disclosure herein, or any other data that may be necessary for carrying out one more processes or methods, or portions or combinations thereof, described herein.

In one or more embodiments, the system **200** may be implemented using one or more computer programs executed on programmable computers, such as computers that include, for example, processing capabilities, data storage (e.g., volatile or non-volatile memory or storage elements), input devices, and output devices. Program code or logic described herein may be applied to input data to perform functionality described herein and generate desired output information. The output information may be applied as input to one or more other devices or methods as described herein or as would be applied in a known fashion.

The program used to implement methods or processes, or portions or combinations thereof, described herein may be provided using any programmable language, e.g., a high level procedural or object orientated programming language that is suitable for communicating with a computer system. Any such programs may, for example, be stored on any suitable device, e.g., a storage media, that is readable by a general or special purpose program running on a computer system (e.g., including processing apparatus) for configuring and operating the computer system when the suitable device is read for performing the procedures described herein. In other words, in embodiments, the system **200** may be implemented using a computer readable storage medium, configured with a computer program, where the storage medium so configured causes the computer to operate in a specific and predefined manner to perform functions described herein. Further, in embodiments, the system **200** may be described as being implemented by logic (e.g., object code) encoded in one or more non-transitory media that includes code for execution and, when executed by a processor, is operable to perform operations such as the methods, processes, or functionality, or portions or combinations thereof, described herein.

Likewise, the system **200** may be configured at a remote site (e.g., an application server) that allows access by one or more users via a remote computer apparatus (e.g., via a web browser), and allows a user to employ the functionality according to the present disclosure (e.g., user accesses a graphical user interface associated with one or more programs to process data).

The computing apparatus **212** may be, for example, any fixed or mobile computer system (e.g., a controller, a microcontroller, a personal computer, mini computer, etc.). The exact configuration of the computing apparatus **212** is not limiting, and essentially any device capable of providing suitable computing capabilities (e.g., graphics processing, etc.) may be used.

As described herein, a digital file may be any medium (e.g., volatile or non-volatile memory, a CD-ROM, a punch card, magnetic recordable tape, etc.) containing digital bits (e.g., encoded in binary, trinary, etc.) that may be readable and/or writeable by computing apparatus **212** described herein.

Also, as described herein, a file in user-readable format may be any representation of data (e.g., ASCII text, binary numbers, hexadecimal numbers, decimal numbers, graphically, etc.) presentable on any medium (e.g., paper, a display, etc.) readable and/or understandable by a user.

In view of the above, it will be readily apparent that the functionality as described in one or more embodiments according to the present disclosure may be implemented in any manner as would be known to one skilled in the art. As such, the computer language, the computer system, or any other software/hardware which is to be used to implement the processes described herein shall not be limiting on the scope of the systems, processes or programs (e.g., the functionality provided by such systems, processes or programs) described herein.

One will recognize that graphical user interfaces may be used in conjunction with the embodiments described herein. The graphical user interfaces may provide various features allowing for user input thereto, change of input, importation or exportation of files, or any other features that may be generally suitable for use with the processes described herein. For example, the graphical user interfaces may allow default values to be used or may require entry of certain values, limits, threshold values, or other pertinent information.

The methods, processes, functionality or logic, or portions or combinations thereof, described in this disclosure, including those attributed to the systems, or various constituent components, may be implemented, at least in part, in hardware, software, firmware, or any combination thereof. For example, various aspects of the techniques may be implemented within one or more processors, including one or more microprocessors, DSPs, ASICs, FPGAs, or any other equivalent integrated or discrete logic circuitry, as well as any combinations of such components, or other devices. The term "processor" or "processing circuitry" may generally refer to any of the foregoing logic circuitry, alone or in combination with other logic circuitry, or any other equivalent circuitry.

Such hardware, software, or firmware may be implemented within the same device or within separate devices to support the various operations and functions described in this disclosure. In addition, any of the described components may be implemented together or separately as discrete but interoperable logic devices. Depiction of different features, e.g., using block diagrams, etc., is intended to highlight different functional aspects and does not necessarily imply that such features must be realized by separate hardware or software components. Rather, functionality may be performed by separate hardware or software components, or integrated within common or separate hardware or software components.

When implemented in software, the functionality ascribed to the systems, devices and methods described in this disclosure may be embodied as instructions and/or logic on a computer-readable medium such as RAM, ROM, NVRAM, EEPROM, FLASH memory, magnetic data storage media, optical data storage media, or the like. The instructions and/or logic may be executed by one or more processors to support one or more aspects of the functionality described in this disclosure.

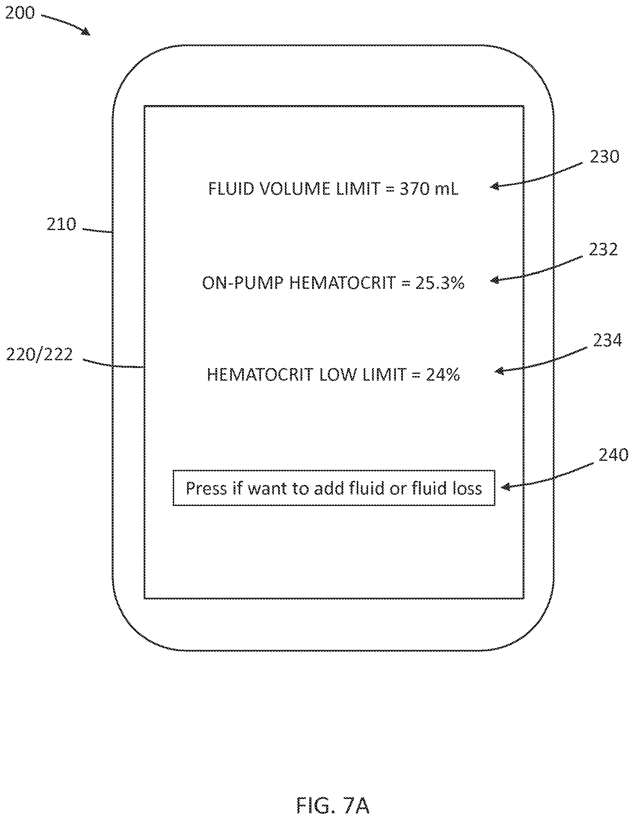

Referring now to **FIGS. 7A-D, 8A-C, 9** and **10A-E,** schematic drawings of an embodiment of a system **200,** such as system **200** depicted in, and described with regard to, **FIG. 6** above, embodied in a device **210** for carrying out one or more method, process, functionality, or logic, or portion or combination thereof, described herein is shown. The depicted device **210** includes a touch screen **220/222** that serves as input apparatus **220** and display apparatus **222.** The touch screen display includes a number of status regions **230, 232, 234, 246, 248, 264** for displaying fluid status information and one or more active region **240, 242, 244, 250, 260, 262, 280, 282, 284, 286, 290, 292.** The active regions are regions configured to display information and receive input. The devices depicted in **FIGS. 7A-D, 8A-C, 9** and **10A-E** are configured to assist a healthcare provider before, during, or after a patient undergoes a CPB procedure.

Referring now to **FIGS. 7A-D,** a sequence of prophetic screen shots are shown that illustrate how a touch screen device **210** may be employed to carry out an embodiment of a method described herein. In **FIG. 7A,** the depicted status regions display a fluid volume limit (**230**), an on-pump hematocrit (**232**), and a set hematocrit low limit (**234**). The information displayed in status regions **230, 232, 234** allow a healthcare provider to better follow a patient's fluid status before, during or after surgery and better enable the healthcare provider to evaluate or understand the effects that fluid addition will have on the patient. As discussed above, the displayed fluid volume limit (in region **230**) indicates the volume of hematocrit-free solution that a patient may receive into the patient's blood compartment to avoid reaching, or to just reach but not fall below, the user defined hematocrit low limit (displayed in region **234**). The on-pump hematocrit is displayed in region **232.** The on-pump hematocrit may be determined and displayed in any suitable manner. For example, the on-pump hematocrit may be calculated, received from a sensor, or input as described above with regard to **FIGS. 2-5.** While not shown, it will be understood that the device **210** may be configured to receive input regarding a patient's weight, hematocrit, desired low limit, etc. for purposes of calculating or determining the displayed information presented in **FIG. 7A**.

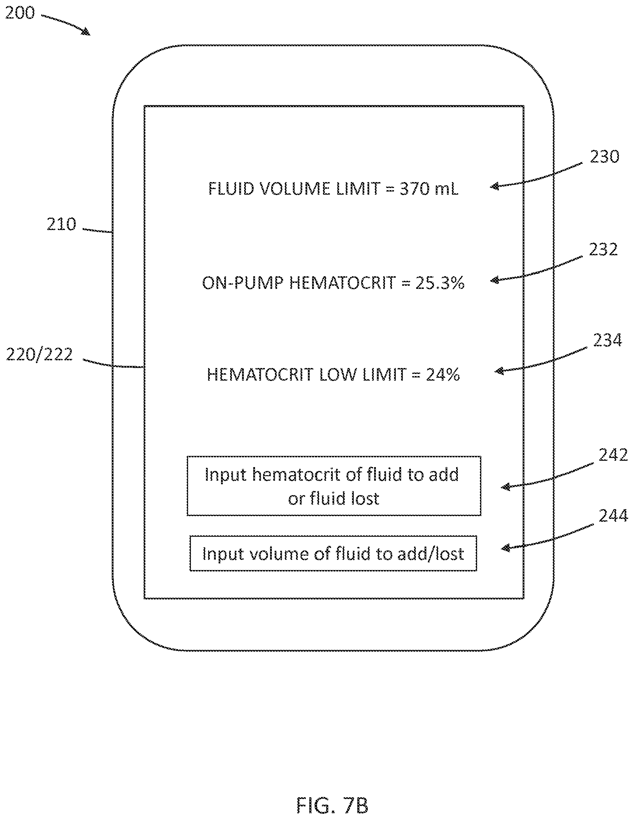

The touchscreen **220/222** of **FIG. 7A** also includes an active region **240.** Active region **240** is configured to display information and receive input; e.g., via touch (finger, stylet, etc.). In the depicted embodiments, active region **240** indicates that a user should touch the active region if fluid is added or lost (or to be added or lost). If the active region is touched, a screen depicted in **FIG. 7B** may result.

**FIG. 7B** includes two active regions **242, 244** configured to display information and receive input. Active region **242** indicates that a user should touch the active region to input hematocrit of fluid added or to be added or lost or to be lost. Active region **244** indicates that a user should touch the active region to input the volume of the fluid added or to be added or lost or to be lost.

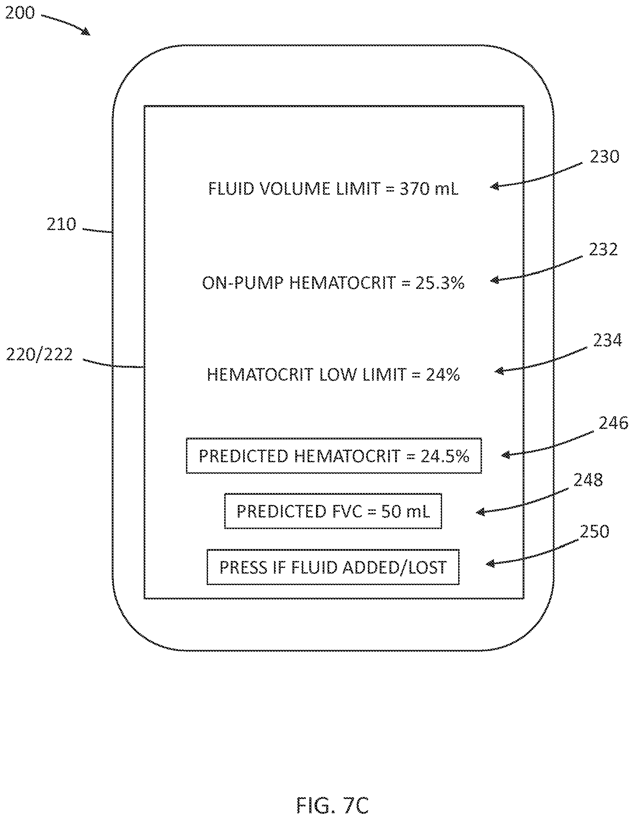

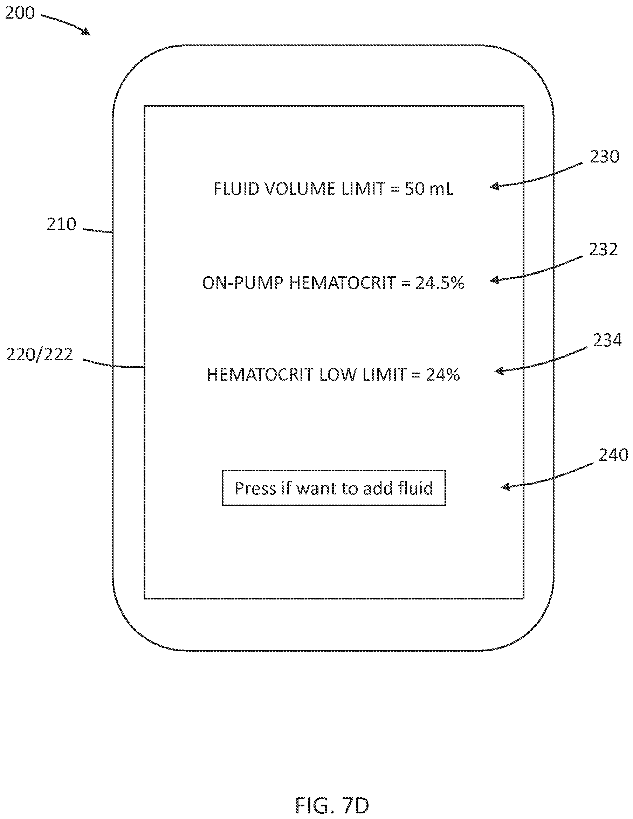

Once the hematocrit and volume of the fluid are added, a screen as depicted in **FIG. 7C** may be presented. In **FIG. 7C,** information regarding predicted hematocrit and FVL are presented in regions **246** and **248,** respectively. Active region **250** indicates that the user should touch the active region if the fluid was actually added or lost. While not shown, it will be understood that a "yes" or "no" option may be chosen by the user. If "no" is chosen, then hematocrit and FVL will not be updated in regions **230** and **232,** and the user will understand what effects the addition of the fluid will have on hematocrit and FVL to better determine whether to add the fluid or adjust one or more parameters of the fluid before adding to the patient's blood fluid compartment. If "yes" is chosen, hematocrit and FVL are updated in regions **230** and **232**; e.g., as shown in **FIG. 7D.**

Also shown in **FIG. 7D** is the return of active region **240,** which allows the user to determine the effect that proposed or actual fluid introduction will have on hematocrit and FVL (e.g., as described above with regard to **FIG. 7A**).

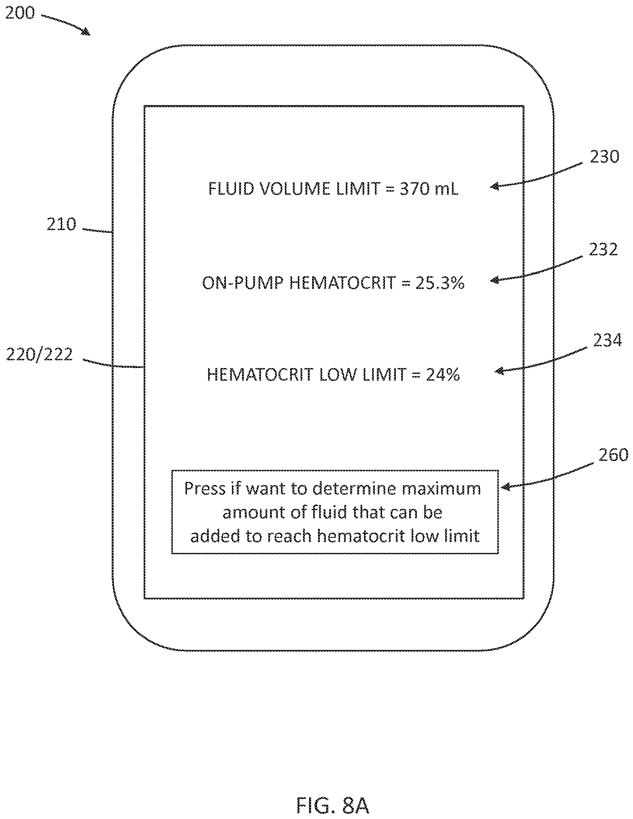

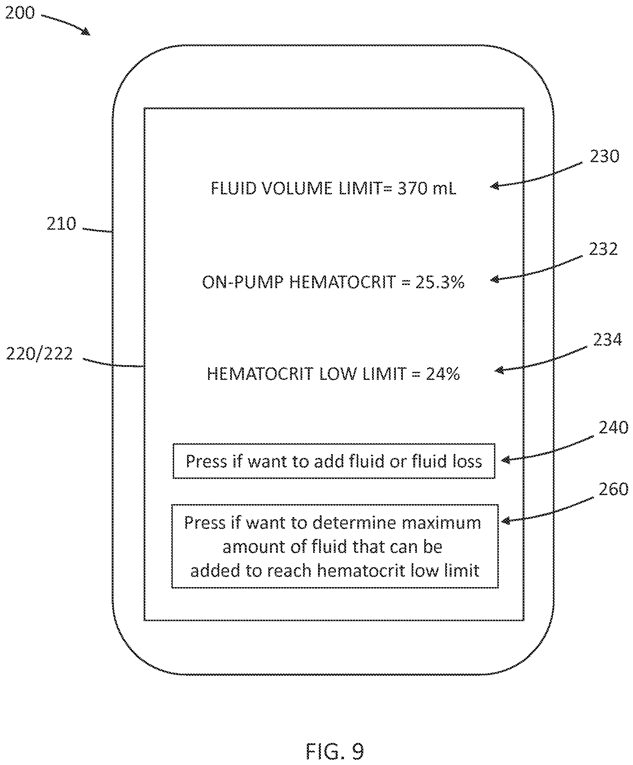

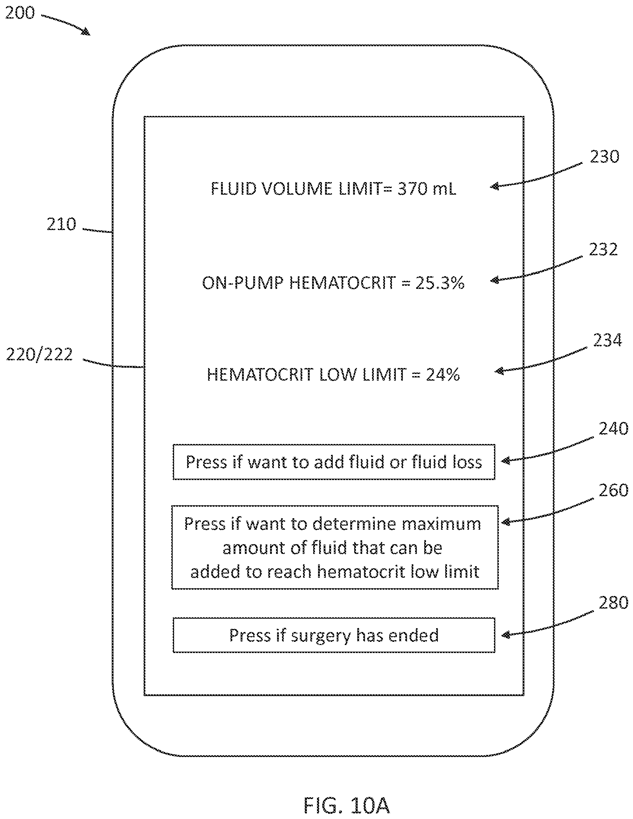

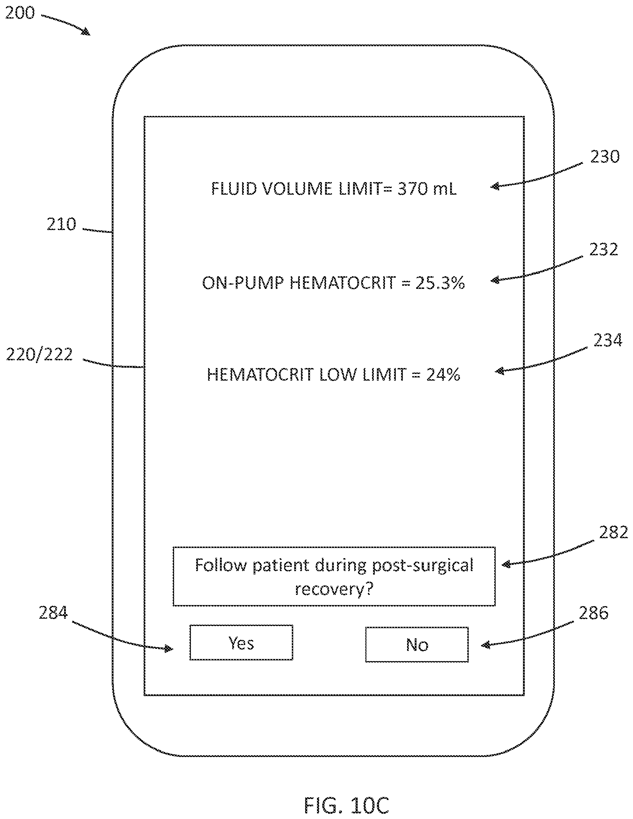

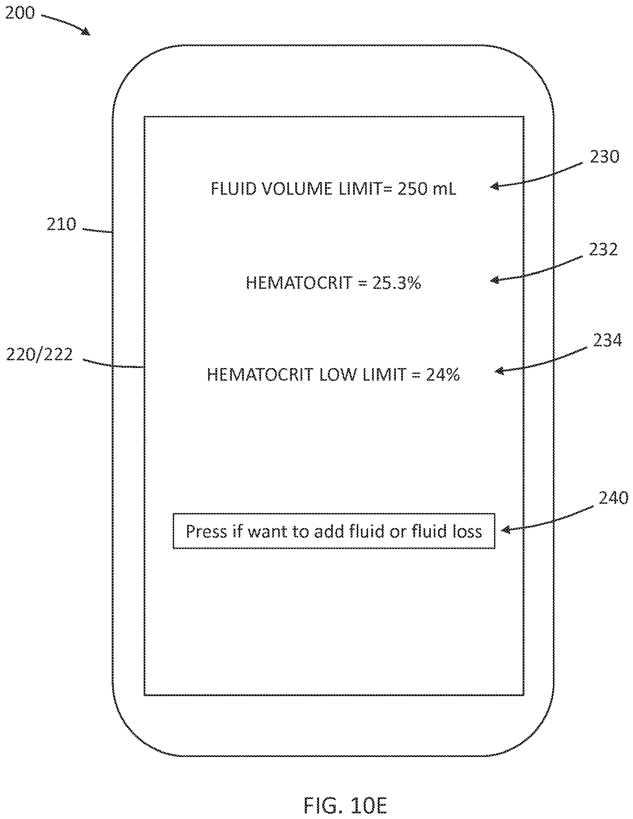

Referring now to **FIGS. 8A-C,** schematic drawings of examples of screen shots of a device configured to carry out an embodiment of a method described herein are shown. The method includes determining the maximum amount of a fluid that can be added to avoid reaching, or to just reach but not fall below, the low limit hematocrit; e.g., as depicted in and described above with regard to **FIG. 5**. The screen shots in **FIGS. 8A-C** (as well as **FIGS. 9** and **10A-E** below) are similar in many respects to the screen shots presented in **FIGS. 7A-D.** For those elements that are not specifically described with regard to **FIGS. 8A-C** (as well as **FIGS. 9** and **10A-E** below), reference is made to discussion of the same or similar elements above with regard to **FIGS. 7A-D.**

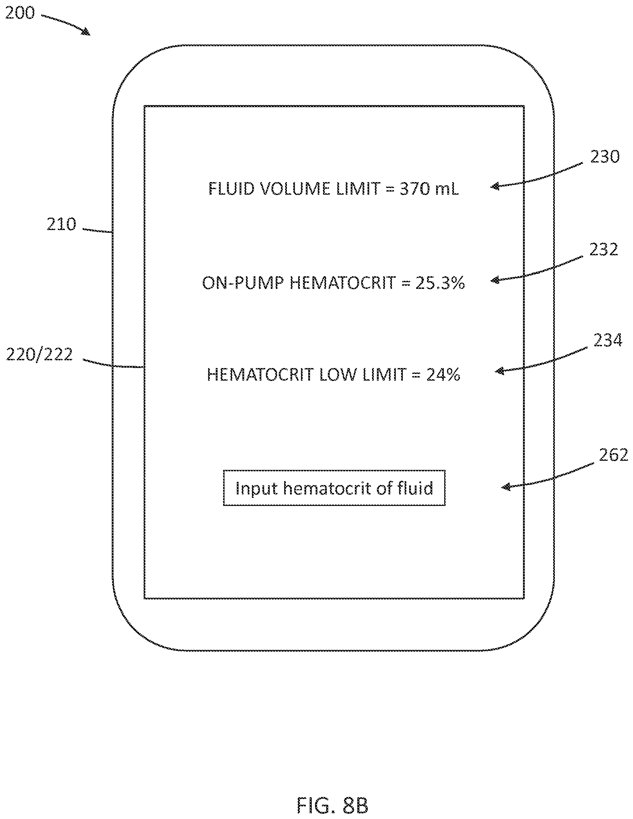

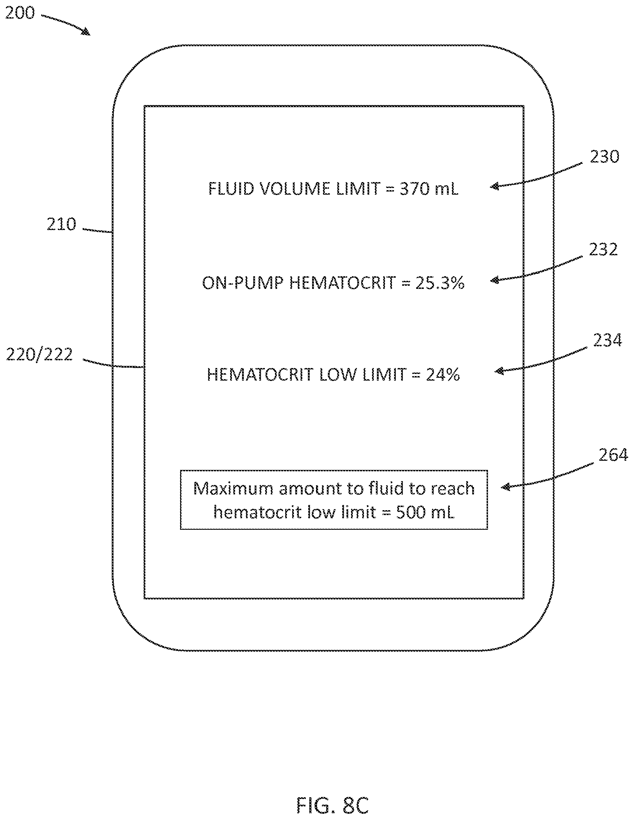

The touchscreen **220/222** in depicted in **FIG. 8A** includes active region **260**. The active region **260** indicates that a user should touch the active region to determine the maximum amount of a fluid that can be added to avoid reaching, or to just reach but not fall below, the user-defined low limit hematocrit. Once active region **260** is touched, active region **262** as depicted in **FIG. 8B** may be presented. Active region **262** indicates that the user should input the hematocrit of the fluid to be administered to the patient's blood fluid compartment. After the hematocrit of the fluid is entered (screen shot not shown), a screen shot as depicted in **FIG. 8C** may result in which region **264** displays the maximum amount of the fluid that may be entered to just reach but not fall below the user-defined hematocrit low limit.

Referring now to **FIG. 9,** a screen shot containing active region **240** as described above with regard to, for example, **FIG. 7A** and active region **260** as described above with regard to **FIG. 8A** is shown. As indicated from **FIG. 9**, the device may be configured to carryout either of the methods associated with **FIGS. 7A-7D** or **FIGS.8A-C.** Of course, a device **210** may be configured to carry out any number of suitable methods described herein, or portions or combinations thereof.

Referring now to **FIGS. 10A-E**, schematic drawings of examples of screen shots of a device configured to carry out an embodiment of a method described herein are shown. The device in **FIGS. 10A-E** provides one example of how device **210** may be used to follow the patient's fluid status during a post-surgical recovery period.

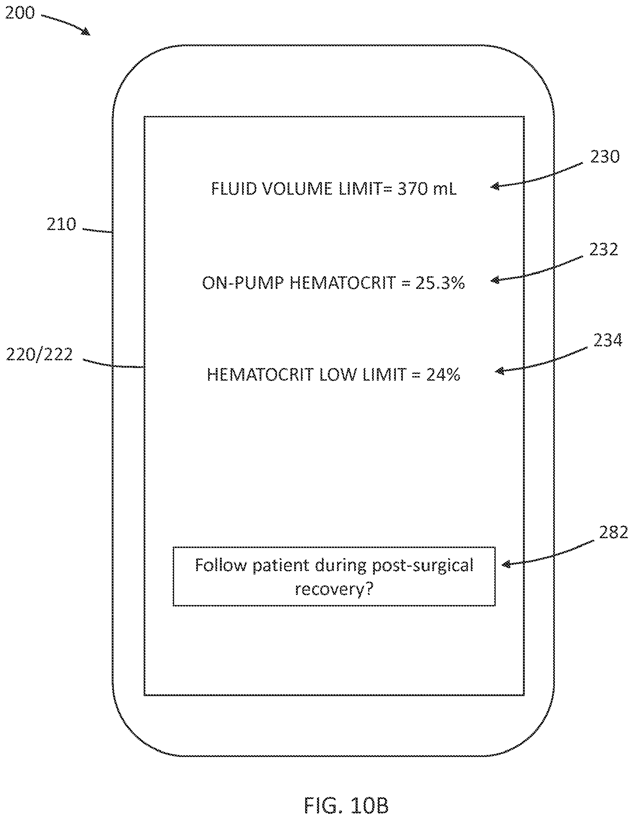

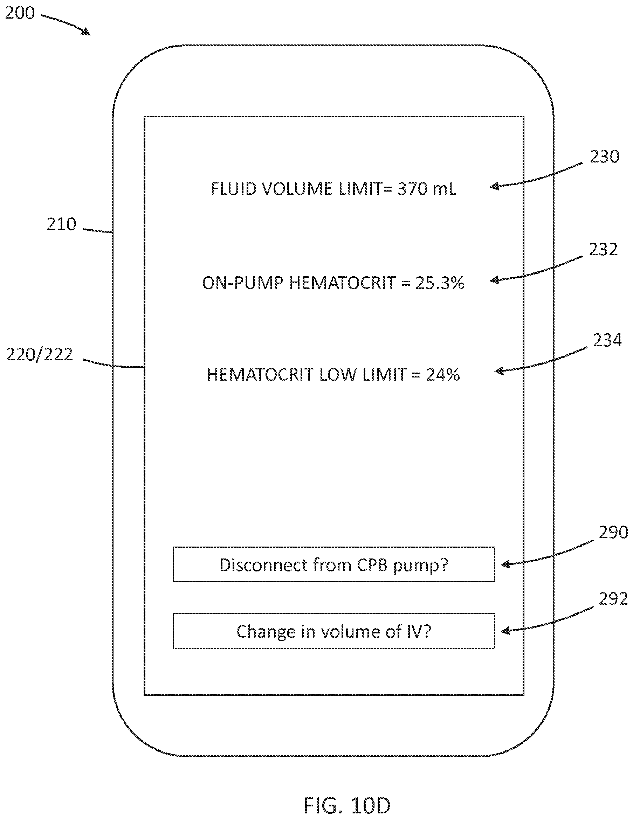

The screen shot depicted in **FIG. 10A** is similar to the screen shot depicted in **FIG. 9. FIG. 10A** includes active region **280** that indicates that a user should touch the active region if the surgery has ended. If active region **280** is touched, active region **282** as depicted in **FIG. 10B** may result. Active region **282** indicates that a user should touch the active region if the user wants to follow the patient's fluid status during post-surgical recovery. If active region **282** is touched, active regions **284** and **286** may be presented to allow a user to verify that, "yes" (**284**), the user would like to follow the patient during post-surgical recovery or to indicate that, "no" (**286**), the user does not want to follow the patient during post-surgical recovery. If active region **284** is touched, active regions **290** and **292** may be presented. Active region **290** indicates that a user should touch the active region if the patient has been disconnected from the CPB pump. The device **210** may calculate effects of removing the CPB prime volume from the blood compartment may have on FVL. Active region **292** indicates that a user should touch the active region to enter any change in IV volume that may have occurred following surgery. The device **210** may calculate effects of changing IV volume on hematocrit and FVL. Any changes in FVL and hematocrit may be presented in regions **230** and **232** and may present active region **240** as depicted in **FIG. 10D** to allow a user to enter any changes in fluid during the patient's recovery.

A number of aspects are presented herein. A summary of some of the selected aspects is presented below.

A first aspect is a computer-implemented method. The method comprises (i) receiving input regarding weight of a patient; (ii) receiving input regarding hematocrit of blood of the patient; (iii) setting a low hematocrit low limit for the patient; and (iv) calculating, based on the input regarding the weight of the patient and the input regarding the hematocrit of the blood of the patient, a volume of hematocrit-free fluid that the patient can receive into a blood compartment of the patient until the low hematocrit limit is reached.

A second aspect is a method of the first aspect, wherein setting the low hematocrit limit comprises receiving input regarding the lower hematocrit limit.

A third aspect is a method of any one of first two aspects, further comprising receiving input regarding a volume and a hematocrit content of a first fluid for introduction into the blood compartment.

A fourth aspect is a method of the third aspect, wherein receiving input regarding the volume and the hematocrit content of the first fluid for introduction into the blood compartment comprises receiving input regarding fluid selected from the group consisting of IV fluid, crystalloid fluid, blood, and a fluid comprising red blood cells.

A fifth aspect is a method of the third aspect or the fourth aspect, further comprising recalculating the volume of hematocrit-free fluid that the patient can receive into the blood compartment of the patient until the low hematocrit limit is reached if the first fluid were introduced into the blood compartment.

A sixth aspect is a method of any one of aspects 3-5, further comprising calculating a volume of the first fluid that can be added to the blood compartment to reach but not fall below the low hematocrit limit.

A seventh aspect is a method of any one of aspects 1-6, further comprising receiving input regarding volume and hematocrit content of fluid loss from a blood compartment of the patient.

An eighth aspect is a method of the seventh aspect, further comprising recalculating the volume of hematocrit-free fluid that the patient can receive into the blood compartment of the patient until the low hematocrit limit is reached based on the input regarding the volume and hematocrit content of the fluid loss.

A ninth aspect is a method of the seventh aspect or the eighth aspect, wherein receiving input regarding the volume and the hematocrit content of the fluid loss from the blood compartment of the patient comprises receiving input regarding fluid loss selected from the group consisting blood loss, urine output, and ultrafiltrate output.

A tenth aspect is a method of any one of aspects 1-9, wherein receiving input regarding hematocrit of blood of the patient comprises receiving input via a sensor.

An eleventh aspect is a method of any one of aspects 1-10, wherein calculating the volume of hematocrit-free fluid that the patient can receive comprises (i) calculating a patient blood volume based on the input regarding the weight of the patient; and (ii) calculating a red cell volume based on the patient blood volume and the input regarding the hematocrit of the patient.

A twelfth aspect is a method of any one of aspects 1-11, further comprising displaying the volume of hematocrit-free fluid that the patient can receive until the lower hematocrit level is reached.

A thirteenth aspect is a computer-implemented method comprising: (i) determining a volume of red blood cell-free fluid that a patient can receive into a blood compartment to reach but not fall below a hematocrit lower limit; (ii) receiving input regarding hematocrit of a first fluid to be added to patient; and (iii) calculating, based on the hematocrit of the first fluid, the volume of the first fluid that can be added to the blood compartment to reach but not fall below the hematocrit lower limit.

A fourteenth aspect is a method of the thirteenth aspect, further comprising displaying the volume of the first fluid that can be added to the blood compartment to reach but not fall below the hematocrit lower limit.

A fifteenth aspect is a non-transitory computer-readable medium comprising instructions that, when implemented, cause a medical device to carry out the method of any one of aspects 1-14.

A sixteenth aspect is a device comprising the non-transitory computer readable medium of the fifteenth aspect.

A seventeenth aspect is a device configured to carry out any one of the methods of aspects 1-14.

In the following, non-limiting prophetic examples are presented of some sample calculations that may be performed in accordance with the methods described above and associated discussion.

**EXAMPLES**

The following prophetic examples, illustrate how formulas regarding fluid volume limit, hematocrit, red blood cell volume, etc. may be used or manipulated to provide fluid volume status of a patient, and associated discussions.

Presented below is an example of how fluid volume limit may be calculated.

(1) Determine the "Patient Blood Volume" (PBV) by multiplying the patient weight (in kilograms) by a 'conversion factor'. This conversion factor represents mL of blood per kilogram of body weight. The value is 65-70.
EXAMPLE: Patient weight = 75 kilograms

$$PBV = 75\,kg \times 70\,mL/kg = 5{,}250\,mL$$

(2) Determine the patient's "Red Cell Volume" (RCV) by multiplying the PBV by the patient's hematocrit.
EXAMPLE: Patient hematocrit = 38%

$$RCV = 5,250\,mL \times 0.38 = 1,995\,mL$$

(3) Establish the user-defined LOW LIMIT HEMATOCRIT (LL-Hct)
EXAMPLE: The team decides that the patient's hematocrit should not drop below 24%.

(4) Determine the FLUID VOLUME LIMIT for this patient.

$$(RCV / LL\text{-}Hct) - PBV = \text{Prime Volume (mL)} + \text{Anesthesia IV Volume}$$

$$(mL)$$

$$(1,995/.24) - 5,250 = 3,063\,mL$$

This means the patient has the capacity to receive a combined total of up to 3,063 mL of crystalloid volume to prevent reaching the user-define LOW LIMIT HEMATOCRIT of 24%.

If the perfusionist uses 1200 mL of crystalloid in the Prime Volume and the anesthesiologist administers 800 mL of IV volume prior to initiating CPB, then the remaining FLUID VOLUME LIMIT after going on CPB is 3,063 mL - 1200 mL - 800 mL = 1,063 mL

The software, hardware, applications (Apps) that carry out the functionality, methods, processes, etc. described herein also have the capability to track fluid additions and fluid losses as they occur during and after cardiac surgery. This includes urine output, ultrafiltrate output ("hemoconcentrator"), blood loss, autologous/cell-washer blood administered, and allogeneic transfusions. The result is a real-time or near real-time upward/downward adjustment in the FLUID VOLUME LIMIT. This allows the team to make pre-emptive decisions relevant to the patient's fluid management status and prevent going below the LOW LIMIT HEMATOCRIT.

When accounting for blood loss, transfusing allogeneic blood products or re-infusing washed red blood cells, it is common practice to only account for the whole volume (in mL) of blood lost or infused. One feature presented herein includes the correction for the RED CELL VOLUME lost or gained to accurately adjust for the FLUID VOLUME LIMIT. If a loss of 300 mL of blood volume occurs with a hematocrit of 29%, this would have a greater impact in the patient's FLUID VOLUME LIMIT than 300 mL of blood loss with a hematocrit of 25%. Here is an example of how this principle is incorporated into the calculations.

For this example, calculations will be based on a 85 kg patient, 70 mL blood volume per kg, and a baseline Hct = 38%.

This makes the patient's blood volume (PBV) = 5,950 mL.

The patient's pre-CPB red cell volume (RCV) = .38 x 5,950 = 2,261 mL

Total pump prime volume added during the case = 1700 mL

Total Anesthesia I.V. Volume added during the case = 1450 mL

The current on-pump Hct = 25.3%

The user-defined low limit hematocrit is 24%.

Using the equation to determine the patient's current "Fluid Volume limit"... (2261/.24) - 5950 -1700 - 1450 = 370 mL.

If the surgical procedure is completed and the patient has been taken off CPB. It is important to realize that once off CPB, it is not a common practice to continuously monitor the patient's hemoglobin or hematocrit levels. These parameters are tracked by periodically sending blood samples to the lab. "Periodically" can be defined to occur every 30-45 minutes, depending on the patient's hemodynamic status and whether there is any bleeding. During this critical period the patient's fluid balance is in a constant state of flux. Because the patient was heparinized during CPB, they will receive Protamine to reverse the anticoagulation effects of heparin. Some residual bleeding may occur around surgical sites while the patient's coagulation status returns to normal. Protamine can induce hemodynamic instability. It is common practice for anesthesia to use fluids in conjunction with inotropic and vasopressor medications to manage cardiovascular hemodynamics.

For bleeding that occurs after coming off CPB, the team will sequester that blood into a separate reservoir to be processed later using a cell washer. For this example, let's assume the perfusionist collects and sequesters 300 mL of blood into the cell washer reservoir. Since it was collected after the time CPB was terminated, it will be assumed the hematocrit of this blood is 25.3%.

At a hematocrit of 25.3%, this 300 mL of blood equates to a loss in the Red Cell Volume (RCV);

$$300 \text{ mL} \times .25.3 = 76 \text{ mL}.$$

There is also a loss of the Patient Blood Volume (PBV), or 300 mL - 76 mL = 224 mL.

The sequence of calculations in this App accommodates these changes as follows; The NEW RCV is 2261 mL - 76 mL = 2,185 mL.

The corrected equation is as follows: (2185/.24) - 5950 - 1700 - 1450 + 224 = 228 mL.

This reduces the Fluid Volume Limit by 142 mL (370 mL - 228 mL). If this same patient had a hematocrit of 29% at the time of the 300 mL blood loss, there occurs a proportionate reduction in Red Cell Volume which then reduces the Fluid Volume Limit down to 171 mL.

Next, consider the situation described above with anesthesia managing the patient's hemodynamics and keeping up with blood loss. Using a traditional fluid management protocol that uses a straight-forward fluid balance calculation (Total Volume IN - Total Volume OUT = FLUID BALANCE), the conventional strategy is to keep up with the mathematical difference and replace the fluid volume lost. In this example, 300 mL of blood loss would means replacing with 300 mL of volume. It is the clinician's discretion as to what kind of volume to use; crystalloid, colloid or allogeneic blood product.

Assume 300 mL of I.V. crystalloid was infused. Based on the calculations used in accordance with the teachings presented herein, 300 mL would exceed the calculated Fluid Volume Limit of 228 mL and possibly cause hematocrit to drop below the user-defined low limit value of 24%.

Using the straight-forward predicted hemodilution algorithm to calculate the effect of infusing 300 mL of crystalloid would lead to a false positive. If loss in RCV is **not** accounted for, the predicted hematocrit after giving 300 mL of crystalloid volume would be as follows (using the uncorrected RCV = 2,261 mL).

$$\text{Predicted Hematocrit} = 2{,}261 \text{ mL} / (5950 + 1700 + 1450 + 300) = .241 \text{ or}$$

$$24.1\%$$

If the loss in RCV **is** accounted for, the predicted hematocrit after giving 300 mL of crystalloid volume would be as follows (using the corrected RCV = 2,185 mL).

$$\text{Predicted Hematocrit} = 2{,}185 \text{ mL} / (5950 + 1700 + 1450 + 300) = .232 \text{ or}$$

$$23.2\%$$

The error occurs by not taking into account the loss in Red Cell Volume.

Accounting for changes in the Red Cell Volume (RCV) is also important when transfusing the patient with processed blood from the Cell Washer. Blood that is sequestered in cell washer reservoir is entered as "unrecovered blood loss". Each time the clinician records a value for the volume of blood sequestered, the software or hardware described herein uses the most recent hematocrit when calculating the new Fluid Volume Limit. Once there is enough volume collected, the clinician will initiate a "wash cycle" to process this sequestered blood. The end-product is washed red blood cells in normal saline. The hematocrit of these washed red cells can range from 45% to 65% because different models of cell washers produce a different quality/concentration of transfusate. The system then transfers the washed red cells into a blood bag for re-infusing to the patient. It is important to note here that, when returning washed red cells, the reliability of the calculation is dependent upon entering a hematocrit value that accurately reflects the blood being returned. While the volume transferred to the blood bag is measured and recorded by the cell washing system, it is at the clinician's discretion to enter a "best guess" value for the hematocrit.

Thus, embodiments of SURGICAL AND POST-SURGICAL FLUID MANAGEMENT are disclosed. One skilled in the art will appreciate that the articles, devices and methods described herein can be practiced with embodiments other than those disclosed. The disclosed embodiments are presented for purposes of illustration and not limitation. One will also understand that components of the devices, systems and methods depicted and described with regard the figures and embodiments herein may be interchangeable.

All scientific and technical terms used herein have meanings commonly used in the art unless otherwise specified. The definitions provided herein are to facilitate understanding of certain terms used frequently herein and are not meant to limit the scope of the present disclosure.

As used in this specification and the appended claims, the singular forms "a", "an", and "the" encompass embodiments having plural referents, unless the content clearly dictates otherwise.

As used in this specification and the appended claims, the term "or" is generally employed in its sense including "and/or" unless the content clearly dictates otherwise. The term "and/or" means one or all of the listed elements or a combination of any two or more of the listed elements.

As used herein, "have", "having", "include", "including", "comprise", "comprising" or the like are used in their open ended sense, and generally mean "including, but not limited to". It will be understood that "consisting essentially of", "consisting of", and the like are subsumed in "comprising" and the like. For example, a method that "comprises" steps A, B, and C may be a method that "consists of" steps A, B and C or that "consists essentially of" steps A, B and C.

As used herein, "consisting essentially of," as it relates to a composition, apparatus, system, method or the like, means that the components of the composition, apparatus, system, method or the like are limited to the enumerated components and any other components that do not materially affect the basic and novel characteristic(s) of the composition, apparatus, system, method or the like.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure, including the claims.

Also herein, the recitations of numerical ranges by endpoints include all numbers subsumed within that range (e.g., 1 to 5 includes 1, 1.5, 2, 2.75, 3, 3.80, 4, 5, etc. or 10 or less includes 10, 9.4, 7.6, 5, 4.3, 2.9, 1.62, 0.3, etc.). Where a range of values is "up to" a particular value, that value is included within the range.

Use of "first," "second," etc. in the description above and the claims that follow is not intended to necessarily indicate that the enumerated number of objects are present. For example, a "second" substrate is merely intended to differentiate from another infusion device (such as a "first" substrate). Use of "first," "second," etc. in the description above and the claims that follow is also not necessarily intended to indicate that one comes earlier in time than the other.

## Claims

1. A computer-implemented method comprising:

   receiving input regarding weight of a patient;
   receiving input regarding hematocrit of blood of the patient;
   setting a low hematocrit limit ($LL_{Hct}$) for the patient;
   calculating a patient's blood volume (PBV) based on the input regarding the weight of the patient; and calculating a red cell volume (RCV) based on the patient's blood volume and the input regarding the hematocrit of the patient, determining an "on-pump" hematocrit of the patient by dividing a red blood cell volume (RCV) by the sum of the patient's blood volume (PBV), a prime volume of a pump (300) and an intravenous volume, i.e. "on-pump" hematocrit of the patient = RCV/(PBV + pump prime volume + IV volume); and
   calculating, based on the input regarding the weight of the patient and the input regarding the hematocrit of the blood of the patient, a volume of hematocrit-free fluid that the patient can receive into a blood compartment of the patient until the low hematocrit limit is reached; and displaying the volume of hematocrit-free fluid that the patient can receive until the lower hematocrit level is reached,
   wherein the volume of hematocrit-free fluid (FVL) is calculated by subtracting the patient's blood volume (PBV), the prime volume of the pump (300) and the intravenous volume from the red blood cell volume (RCV) divided by the set hematocrit low limit ($LL_{Hct}$), i.e. FVL = (RCV/$LL_{Hct}$) - PBV - pump prime volume - IV volume.

2. The method of claim 1, wherein setting the low hematocrit limit comprises receiving input regarding the lower hematocrit limit.

3. The method of claim 1, further comprising receiving input regarding a volume and a hematocrit content of a first fluid for introduction into the blood compartment.

4. The method of claim 3, wherein receiving input regarding the volume and the hematocrit content of the first fluid for introduction into the blood compartment comprises receiving input regarding fluid selected from the group consisting of IV fluid, crystalloid fluid, blood, and a fluid comprising red blood cells, or further comprising recalculating the volume of hematocrit-free fluid that the patient can receive into the blood compartment of the patient until the low hematocrit limit is reached if the first fluid were introduced into the blood compartment, or further comprising calculating a volume of the first fluid that can be added to the blood compartment to reach but not fall below the low hematocrit limit.

5. The method of claim 1, further comprising receiving input regarding volume and hematocrit content of fluid loss from a blood compartment of the patient.

6. The method of claim 5, further comprising recalculating the volume of hematocrit-free fluid that the patient can receive into the blood compartment of the patient until the low hematocrit limit is reached based on the input regarding the volume and hematocrit content of the fluid loss.

7. The method of claim5, wherein receiving input regarding the volume and the hematocrit content of the fluid loss from the blood compartment of the patient comprises receiving input regarding fluid loss selected from the group consisting blood loss, urine output, and ultrafiltrate output.

8. The method of claim 1, wherein receiving input regarding hematocrit of blood of the patient comprises receiving input via a sensor.

9. A non-transitory computer-readable medium comprising instructions that, when implemented, cause a medical device to carry out the method of claim 1.

10. A device comprising the non-transitory computer readable medium of claim 9.

11. A device configured to carry out any one of the methods of claim 1.


**Patentansprüche**

1. Computerimplementiertes Verfahren, umfassend:

Empfangen einer Eingabe hinsichtlich des Gewichts eines Patienten;
Empfangen einer Eingabe hinsichtlich eines Hämatokritwerts von Blut des Patienten;
Festlegen eines Grenzwerts für niedrige Hämatokritwerte ($LL_{Hct}$) für den Patienten;
Berechnen des Blutvolumens eines Patienten (PBV) auf Grundlage der Eingabe hinsichtlich des Gewichts des Patienten; und
Berechnen eines Erythrozytenvolumens (RCV) auf Grundlage des Blutvolumens des Patienten und der Eingabe hinsichtlich des Hämatokritwerts des Patienten,
Bestimmen eines Hämatokritwerts des Patienten "mit Herz-Lungen-Maschine" durch Dividieren eines Erythrozytenvolumens (RCV) durch die Summe des Blutvolumens des Patienten (PBV), eines Füllvolumens einer Pumpe (300) und eines intravenösen Volumens, d. h. Hämatokritwert des Patienten "mit Herz-Lungen-Maschine" = RCV/(PBV + Füllvolumen der Pumpe + IV-Volumen); und
Berechnen eines Volumens von hämatokritfreier Flüssigkeit, das der Patient in ein Blutkompartiment des Patienten aufnehmen kann, bis der Grenzwert für niedrige Hämatokritwerte erreicht ist, auf Grundlage der Eingabe hinsichtlich des Gewichts des Patienten und der Eingabe hinsichtlich des Hämatokritwerts des Bluts des Patienten; und
Anzeigen des Volumens von hämatokritfreier Flüssigkeit, das der Patient aufnehmen kann, bis der untere Hämatokritspiegel erreicht ist,
wobei das Volumen von hämatokritfreier Flüssigkeit (FVL) durch Subtrahieren des Blutvolumens des Patienten (PBV), des Füllvolumens der Pumpe (300) und des intravenösen Volumens von dem Erythrozytenvolumen (RCV) dividiert durch den festgelegten Grenzwert für niedrige Hämatokritwerte ($LL_{Hct}$) berechnet wird, d. h. FVL = ($RCV/LL_{Hct}$) - PBV - Füllvolumen der Pumpe - IV-Volumen.

2. Verfahren nach Anspruch 1, wobei Festlegen des Grenzwerts für niedrige Hämatokritwerte Empfangen einer Eingabe hinsichtlich des unteren Hämatokritgrenzwerts umfasst.

3. Verfahren nach Anspruch 1, ferner umfassend Empfangen einer Eingabe hinsichtlich eines Volumens und eines Hämatokritgehalts einer ersten Flüssigkeit zur Einbringung in das Blutkompartiment.

4. Verfahren nach Anspruch 3, wobei Empfangen einer Eingabe hinsichtlich des Volumens und des Hämatokritgehalts der ersten Flüssigkeit zur Einbringung in das Blutkompartiment Empfangen einer Eingabe hinsichtlich einer Flüssigkeit umfasst, die aus der Gruppe ausgewählt ist, die aus IV-Flüssigkeit, kristalloider Flüssigkeit, Blut und einer Flüssigkeit, die rote Blutkörperchen umfasst, besteht, oder ferner umfassend erneutes Berechnen des Volumens von hämatokritfreier Flüssigkeit, das der Patient in das Blutkompartiment des Patienten aufnehmen kann, bis der Grenzwert für niedrige Hämatokritwerte erreicht ist, falls die erste Flüssigkeit in das Blutkompartiment eingebracht würde, oder ferner umfassend Berechnen eines Volumens der ersten Flüssigkeit, das in das Blutkompartiment hinzugegeben werden kann, um den Grenzwert für niedrige Hämatokritwerte zu erreichen, aber nicht unter diesen zu fallen.

5. Verfahren nach Anspruch 1, ferner umfassend Empfangen einer Eingabe hinsichtlich des Volumens und Hämatokritgehalts von Flüssigkeitsverlust aus einem Blutkompartiment des Patienten.

6. Verfahren nach Anspruch 5, ferner umfassend erneutes Berechnen des Volumens von hämatokritfreier Flüssigkeit, das der Patient in das Blutkompartiment des Patienten aufnehmen kann, bis der Grenzwert für niedrige Hämatokritwerte erreicht ist, auf Grundlage der Eingabe hinsichtlich des Volumens und Hämatokritgehalts des Flüssigkeitsverlusts.

7. Verfahren nach Anspruch 5, wobei Empfangen einer Eingabe hinsichtlich des Volumens und des Hämatokritgehalts des Flüssigkeitsverlusts aus dem Blutkompartiment des Patienten Empfangen einer Eingabe hinsichtlich Flüssigkeitsverlusts umfasst, der aus der Gruppe ausgewählt ist, die aus Blutverlust, Urinausscheidung und Ultrafiltratausscheidung besteht.

8. Verfahren nach Anspruch 1, wobei Empfangen einer Eingabe hinsichtlich eines Hämatokritwerts von Blut des Patienten Empfangen einer Eingabe über einen Sensor umfasst.

9. Nichtflüchtiges computerlesbares Medium, das Anweisungen umfasst, die bei Implementierung bewirken, dass eine medizinische Vorrichtung das Verfahren nach Anspruch 1 ausführt.

10. Vorrichtung, die das nichtflüchtige computerlesbare Medium nach Anspruch 9 umfasst.

11. Vorrichtung, die dazu konfiguriert ist, ein beliebiges der Verfahren nach Anspruch 1 auszuführen.

**Revendications**

1. Procédé implémenté par ordinateur comprenant :

la réception d'une entrée concernant le poids d'un patient ;
la réception d'une entrée concernant l'hématocrite du sang du patient ;
l'établissement d'une limite basse d'hématocrite ($LL_{Hct}$) pour le patient ;
le calcul d'un volume sanguin du patient (PBV) sur la base de l'entrée concernant le poids du patient ; et le calcul d'un volume de globule rouge (RCV) sur la base du volume sanguin du patient et de l'entrée concernant l'hématocrite du patient,
la détermination d'un hématocrite « sur pompe » du patient en divisant un volume de globule rouge (RCV) par la somme du volume sanguin du patient (PBV), d'un volume d'amorçage d'une pompe (300) et d'un volume intraveineux, c'est-à-dire qu'un hématocrite « sur pompe » du patient = RCV/(PBV + volume d'amorçage de pompe + volume IV) ; et
le calcul, sur la base de l'entrée concernant le poids du patient et de l'entrée concernant l'hématocrite du sang du patient, d'un volume de fluide exempt d'hématocrite que le patient peut recevoir dans un compartiment sanguin du patient jusqu'à ce que la limite basse d'hématocrite soit atteinte ; et
l'affichage du volume de fluide exempt d'hématocrite que le patient peut recevoir jusqu'à ce que le niveau inférieur d'hématocrite soit atteint,
dans lequel le volume de fluide exempt d'hématocrite (FVL) est calculé en soustrayant le volume sanguin du patient (PBV), le volume d'amorçage de la pompe (300) et le volume intraveineux du volume de globule rouge (RCV) divisé par la limite basse d'hématocrite ($LL_{Hct}$) établie, c'est-à-dire FVL = ($RCV/LL_{Hct}$) - PBV - volume d'amorçage de pompe - volume IV.

2. Procédé selon la revendication 1, dans lequel l'établissement de la limite basse d'hématocrite comprend la réception d'une entrée concernant la limite inférieure d'hématocrite.

3. Procédé selon la revendication 1, comprenant en outre la réception d'une entrée concernant un volume et une teneur en hématocrite d'un premier fluide pour introduction dans le compartiment sanguin.

4. Procédé selon la revendication 3, dans lequel la réception d'une entrée concernant le volume et la teneur en hématocrite du premier fluide pour introduction dans le compartiment sanguin comprend la réception d'une entrée concernant un fluide sélectionné dans le groupe constitué d'un fluide IV, d'un fluide cristalloïde, du sang, et d'un fluide comprenant des globules rouges, ou comprenant en outre le recalcul du volume de fluide exempt d'hématocrite que le patient peut recevoir dans le compartiment sanguin du patient jusqu'à ce que la limite basse d'hématocrite soit atteinte si le premier fluide a été introduit dans le compartiment sanguin, ou comprenant en outre le calcul d'un volume du premier fluide qui peut être ajouté au compartiment sanguin pour atteindre la limite basse d'hématocrite mais sans tomber en dessous.

5. Procédé selon la revendication 1, comprenant en outre la réception d'une entrée concernant un volume et une teneur en hématocrite d'une perte de fluide à partir d'un compartiment sanguin du patient.

6. Procédé selon la revendication 5, comprenant en outre le recalcul du volume de fluide exempt d'hématocrite que le patient peut recevoir dans le compartiment sanguin du patient jusqu'à ce que la limite basse d'hématocrite soit atteinte sur la base de l'entrée concernant le volume et la teneur en hématocrite de la perte de fluide.

7. Procédé selon la revendication 5, dans lequel le réception d'une entrée concernant le volume et la teneur en hématocrite de la perte de fluide à partir du compartiment sanguin du patient comprend la réception d'une entrée concernant une perte de fluide sélectionnée dans le groupe constitué d'une perte de sang, d'une sortie d'urine, et d'une sortie d'ultrafiltrat.

8. Procédé selon la revendication 1, dans lequel la réception d'une entrée concernant l'hématocrite du sang du patient comprend la réception d'une entrée via un capteur.

9. Support non transitoire lisible par ordinateur comprenant des instructions qui, quand elles sont implémentées, amènent un dispositif médical à mettre en œuvre le procédé selon la revendication 1.

10. Dispositif comprenant le support non transitoire lisible par ordinateur selon la revendication 9.

11. Dispositif configuré pour mettre en œuvre l'un quelconque des procédés selon la revendication 1.

FIG. 1

Input patient weight — 10

30 — Input hematocrit → Display Hct — 35

Determine patient blood volume
(weight x conversion factor)

20

Set hematocrit low limit

40

50 — Determine red cell volume
(PVB x hematocrit)

Display LL$_{Hct}$ — 45

Determine fluid volume limit
[(RCV/LL$_{Hct}$) – PBV] — 60

Display fluid volume limit — 65

FIG. 2

```
                                        ┌─────────────────────────────┐
                                        │  Determine fluid volume limit │ ⟋ 60
                                        │    [(RCV/LL_Hct) − PBV]        │
                                        └──────────────┬──────────────┘
                                                       │
   ┌──────────────────────────────┐                  │
70 │   Input volume of RBC-free fluid├──────────────────┤
   │       added/to be added       │                  │
   └──────────────┬───────────────┘                  │
                  │                                    │
                  ▼                                    ▼
   ┌──────────────────────────┐      ┌────────────────────────────────────┐
75 │   Determine Hct           │      │     Determine adjusted FVL          │ ⟋ 80
   │  [RCV/(PBV + V_add)]      │      │  [(RCV/LL_Hct) − PBV − V_RBC-free]  │
   └──────────────┬───────────┘      └────────────────┬───────────────────┘
                  │                                    │
                  ▼                                    ▼
37 ┌──────────────────────┐          ┌────────────────────────────┐
   │    Display  Hct       │          │    Display  adjusted fluid  │ ⟋ 85
   └──────────▲───────────┘          │       volume limit          │
              │                       └────────────────────────────┘
30 ┌──────────────────────┐
   │     Input Hct         │
   └──────────────────────┘
```

FIG. 3

Input volume and Hct of RBC-containing fluid added/to be added — 90

77 — Determine adjusted Hct
$$[Hct_{new} = Hct_{prev} (PBV_{prev} /PBV_{new}) + Hct_{add} (V_{add}/PBV_{new})]$$

Determine adjusted patient blood volume ($PBV_{prev} + V_{add} = PBV_{new}$) — 100

37 — Display Hct

30 — Input Hct

Determine adjusted red blood cell volume ($RVC_{new} = PBV_{new} \times Hct_{new}$) — 110

Determine adjusted FVL
$$[(RCV_{new}/LL_{Hct}) - PBV_{new}]$$ — 120

Display adjusted fluid volume limit — 125

FIG. 4

20

Determine fluid volume limit
$[(RCV/LL_{Hct}) - PBV]$ — 60

Input volume and Hct
fluid to be added — 90

Determine amount of fluid that
can be added until $LL_{Hct}$ reached — 130

Display amount that can
be added — 135

FIG. 5

FIG. 6

200

230

FLUID VOLUME LIMIT = 370 mL

210

ON-PUMP HEMATOCRIT = 25.3%

232

220/222

HEMATOCRIT LOW LIMIT = 24%

234

240

Press if want to add fluid or fluid loss

FIG. 7A

FIG. 7B

200

210

220/222

FLUID VOLUME LIMIT = 370 mL — 230

ON-PUMP HEMATOCRIT = 25.3% — 232

HEMATOCRIT LOW LIMIT = 24% — 234

PREDICTED HEMATOCRIT = 24.5% — 246

PREDICTED FVC = 50 mL — 248

PRESS IF FLUID ADDED/LOST — 250

FIG. 7C

200

210

220/222

230

FLUID VOLUME LIMIT = 50 mL

232

ON-PUMP HEMATOCRIT = 24.5%

234

HEMATOCRIT LOW LIMIT = 24%

240

Press if want to add fluid

FIG. 7D

26

200

210

220/222

230
FLUID VOLUME LIMIT = 370 mL

232
ON-PUMP HEMATOCRIT = 25.3%

234
HEMATOCRIT LOW LIMIT = 24%

260
Press if want to determine maximum
amount of fluid that can be
added to reach hematocrit low limit

FIG. 8A

200

230

FLUID VOLUME LIMIT = 370 mL

210

232

ON-PUMP HEMATOCRIT = 25.3%

220/222

234

HEMATOCRIT LOW LIMIT = 24%

262

Input hematocrit of fluid

FIG. 8B

FLUID VOLUME LIMIT = 370 mL — 230

ON-PUMP HEMATOCRIT = 25.3% — 232

HEMATOCRIT LOW LIMIT = 24% — 234

Maximum amount to fluid to reach
hematocrit low limit = 500 mL — 264

200

210

220/222

FIG. 8C

200

210

220/222

230

FLUID VOLUME LIMIT= 370 mL

232

ON-PUMP HEMATOCRIT = 25.3%

234

HEMATOCRIT LOW LIMIT = 24%

240

Press if want to add fluid or fluid loss

260

Press if want to determine maximum
amount of fluid that can be
added to reach hematocrit low limit

FIG. 9

200

230
FLUID VOLUME LIMIT= 370 mL

210

232
ON-PUMP HEMATOCRIT = 25.3%

220/222

234
HEMATOCRIT LOW LIMIT = 24%

240
Press if want to add fluid or fluid loss

260
Press if want to determine maximum
amount of fluid that can be
added to reach hematocrit low limit

280
Press if surgery has ended

FIG. 10A

200

210

220/222

FLUID VOLUME LIMIT= 370 mL

230

ON-PUMP HEMATOCRIT = 25.3%

232

HEMATOCRIT LOW LIMIT = 24%

234

Follow patient during post-surgical recovery?

282

FIG. 10B

200

230

FLUID VOLUME LIMIT= 370 mL

232

ON-PUMP HEMATOCRIT = 25.3%

210

234

220/222

HEMATOCRIT LOW LIMIT = 24%

282

Follow patient during post-surgical recovery?

284

Yes

No

286

FIG. 10C

FLUID VOLUME LIMIT= 370 mL

ON-PUMP HEMATOCRIT = 25.3%

HEMATOCRIT LOW LIMIT = 24%

Disconnect from CPB pump?

Change in volume of IV?

FIG. 10D

FLUID VOLUME LIMIT= 250 mL    230

210

HEMATOCRIT = 25.3%    232

220/222    HEMATOCRIT LOW LIMIT = 24%    234

240
Press if want to add fluid or fluid loss

200

FIG. 10E

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2581846 A1
- US 2010152544 A1
- US 5984893 A